# EUROPÄISCHE PATENTSCHRIFT

(11) **EP 1 096 918 B1**
(45) Veröffentlichungstag und Bekanntmachung des Hinweises auf die Patenterteilung: **09.11.2005**
(21) Anmeldenummer: 99947221.0
(22) Anmeldetag: 15.07.1999
(51) Int. Cl.: A61K 7/48, A23L 1/304, A23L 1/305, A61K 33/40, A61K 33/30

(54) **MITTEL ZUR PFLEGE UND/ODER BEHANDLUNG VON HAUT UND GEWEBE**
SKIN AND TISSUE CARE AND/OR TREATMENT AGENT
AGENT DE SOIN ET/OU DE TRAITEMENT DE LA PEAU ET DE TISSUS

(30) Priorität: 15.07.1998 DE 19831798
(43) Veröffentlichungstag der Anmeldung: 09.05.2001
(73) Patentinhaber: Mandorlo Investment GmbH, 2210 Luxembourg (LU)
(72) Erfinder: SCHLACHTER, Herbert, 85716 Lohhof-Unterschleissheim (DE)
(74) Vertreter: Vossius & Partner
(86) Internationale Anmeldenummer: PCT/DE1999/002202
(87) Internationale Veröffentlichungsnummer: WO 2000/003689

(56) Entgegenhaltungen:
- EP-A- 0 281 812
- EP-A- 0 385 155
- EP-A- 0 414 605
- EP-A- 0 714 655
- WO-A-91/11117
- WO-A-98/10741
- DE-B- 2 840 498
- FR-A- 2 674 755
- US-A- 4 283 386
- US-A- 5 597 585
- US-A- 5 681 852

## Beschreibung

Die Erfindung betrifft ein Mittel zur Pflege und/oder Behandlung von Haut und Gewebe. Insbesondere betrifft die Erfindung ein Mittel zur Pflege, zum Schutz, zur Vorbeugung gewebsschädigender Manifestationen und Einwirkungen und zur Behandlung von Haut und Gewebe, welches mindestens ein Salz ausgewählt aus Alkali- und Erdalkalimetallsalzen und anderen Mineralstoffen, umfaßt, dadurch gekennzeichnet, daß es mindestens eine Aminosäure und Zinkoxid und/oder ein anorganisches Peroxid, vorzugsweise Magnesium- öder Natriumperoxid, enthält. Das erfindungsgemäße Mittel kann zusätzlich gegebenenfalls ein adstringierendes Mittel, ein Binde- und Haftmittel, ein Feuchtigkeitsmittel, ein etherisches Öl, Tego-Betain, sekundäre Pflanzenstoffe, wie Epigallocatechine, ungesättigte Fettsäuren, Liposomen, Vitamine, Spurenelemente und antimykotische und/oder antimikrobische Komponenten umfassen.

Natürliche Nährstoffe sind die Voraussetzung für die Gesundheit von Zellen und des Körpers. Sie dienen der Erhaltung und Regeneration der Haut, sowie der Förderung des Stoffwechsels und der Sauerstoffversorgung der Zellen. Diese umfassende Bedeutung der Nährstoffversorgung rückt auch im Bereich von Hautpflege, Kosmetik und Dermatologie immer mehr in den Vordergrund. Hormonelle Veränderungen, Veranlagung, einseitige und falsche Ernährung und der Gesundheit abträgliche Lebensgewohnheiten, insbesondere Rauchen und Bewegungsarmut, führen zu typischen Erscheinungsbildern der Haut, wie Gewebsveränderungen, Gewebsdeformationen und zellulären Stoffwechselstörungen. Bei gezielter Zufuhr von Nährstoffen kommt es zur Harmonisierung des Stoffwechsels und somit zun natürlichen, physiologischen Gleichgewicht der Zellen.

Es ist allgemein bekannt, daß Vitamine, Mineralstoffe und Spurenelemente für die Nährstoffversorgung der Haut unerläßlich sind. Eiweiße bzw. Proteine, die aus Aminosäuren aufgebaut sind, sind ein wichtiger Aufbaustoff für Zellen und körpereigene Wirkstoffe, wie Enzyme und bestimmte Hormone. Zunehmend gewinnen auch mehrfach ungesättigte Fettsäuren, sekundäre Pflanzenstoffe wie die Flavonoide und Epigallocatechine, und Liposomen allgemein für die Gesundheit und Leistungsfähigkeit an Bedeutung. Vitamine, Mineralstoffe und Spurenelemente, mehrfach ungesättigte Fettsäuren und bioaktive Pflanzenstoffe wie z.B. Flavonoide, sind lebensnotwendige Reglerstoffe im Stoffwechsel und Schutznährstoffe für die Gesundheit der Haut.

Vitamine sind lebensnotwendige Nahrungsbestandteile, die für die normalen Funktionen heterotropher Lebewesen mehr oder weniger obligat und bedarfsgerecht zuzuführen sind, da sie nur aus äußeren Quellen bzw. unter dem Einfluß von Millieufaktoren (z.B. Darmflora) zugänglich sind. Ihre spezifische biokatalytische Wirkung beruht auf dem Ersatz der dem metabolischen Verschleiß unterliegenden Wirkgruppen von Enzymen. Von wissenschaftlicher Seite her ist z.B. bekannt, daß die Vitamine der B-Gruppe als Coenzym am intermediären Stoffwechsel beteiligt sind und die Vitamine C, E und β-Carotin vor allem als Antioxidantien wirken. Mangel infolge ungenügender Zufuhr oder Resorption, Störungen der Darmflora oder Metabolismus, Antivitamin-Einwirkung oder gesteigerter Verbrauch führen zu Hypo- und Avitaminosen.

Mineralstoffe und Spurenelemente sind ferner lebensnotwendige Reglerstoffe im Stoffwechsel. Zink, Magnesium und die Vitamine der B-Gruppe sind Hochleistungselemente, indem sie Enzyme aktivieren und den Stoffwechsel von Kohlenhydraten, Fetten und Eiweißstoffen erst ermöglichen. Silicium wirkt sich günstig auf die Stabilität und den Erhalt der Haut aus. Ferner ist wissenschaftlich unbestritten, daß Zink eine essentielle Funktion im Immunsystem und im Stoffwechsel der Haut hat.

Mehrfach ungesättigte Fettsäuren enthalten Linolsäure und α- und γ-Linolensäure, die wichtige Ausgangssubstanzen für biologisch aktive Reglersubstanzen, wie z.B. Eicosanoide und Prostaglandine, im Stoffwechsel sind, und die für ein gesundes Gleichgewicht im Stoffwechsel sorgen. Eicosanoide und Prostaglandine, auch als Gewebshormone bezeichnet, werden von Wissenschaftlern in bezug auf ihre gesundheitsstabilisierende Wirkung zur Zeit intensiv erforscht. Bekannt ist der günstige Einfluß der mehrfach ungesättigten Fettsäuren auf die gesunde Hautfunktion sowie bei entzündlichen Vorgängen. Ferner ist bekannt, daß mehrfach ungesättigte Fettsäuren vom Typ der ω-3-(Eicosapentaensäure, α-Linolensäure) und ω-6-Fettsäuren (Linolsäure, γ-Linolensäure) günstige Effekte bei z.B. Neurodermitis und Psoriasis sowie bei belastungsinduzierten Regenerationsprozessen haben. Die Bedeutung der sogenannten sekundären Pflanzenstoffe, fachwissenschaftlich auch bioaktive Pflanzenstoffe genannt, für die Gesundheit wird zur Zeit ebenfalls erforscht. Zu diesen natürlichen Pflanzenstoffen zählen auch Bioflavonoide, die die Wirkung von Vitamin C in bezug auf die Abwehrkräfte, die Gefäßwände und das Bindegewebe wirksam unterstützen. Ferner ist bekannt, daß die Bioflavonoide antioxidative Eigenschaften haben und damit die Wirkung der Vitamine C, E und β-Carotin synergistisch ergänzen.

Aminosäuren werden nach Biosynthese-Aspekten in essentielle, semi-essentielle und nicht-essentielle Aminosäuren unterschieden. Aminosäuren sind neben ihrer Funktion als Proteinbausteine Vorstufen von biologisch wirksamen Verbindungen. Aminosäuren sind u.a. beschrieben in L. Stryer, Biochemie, Spektrum Akademischer Verlag, Oxford, 1994 und in Römpp Lexikon Chemie, Herausgeber J. Falbe und M. Regitz, Stichwort "Aminosäuren", Thieme Verlag, 1996 und darin zitierte Literaturstellen.

Liposomen sind von ein- oder mehrschichtigen Phospholipid-Doppelmembranen umgebende Partikel, die in der inneren, wäßrigen Phase mit hydrophilen Arzneistoffmolekülen beladen werden können. Mit dem Einsatz als Arzneistoffträger kann eine gezielte lokale Anreicherung von Wirkstoffen und verzögerte Wirkstoffabgabe erreicht werden.

Im Handel sind zahlreiche Mittel zur Behandlung und Vorbeugung von mykotischen, mikrobischen, pathologischen und sonstigen gewebsschädigenden Manifestationen und Einwirkungen, Gewebsveränderungen, Gewebsdeformationen, zellulären Stoffwechselstörungen und sonstigen Verletzungen von menschlichem Gewebe erhältlich. Beispielsweise gibt es verschiedenste Behandlungsansätze gegen Haut- und Bindegewebsprobleme. Viele dieser Mittel weisen jedoch eine zweifelhafte Erfolgsbilanz auf, da es diesen Mitteln nicht möglich ist, bis zu den geschädigten Zellen vorzudringen und/oder in den geschädigten Zellen zu wirken. Ferner berücksichtigen viele der im Handel erhältlichen Produkte nicht den von Wissenschaftlern entdeckten Wirkmechanismus, indem die Zelle auf natürliche Weise remineralisiert und der Stoffwechsel angeregt wird, so daß sich der natürliche Zellinnendruck wieder herstellt, das Zellvolumen normalisiert und Fett und Schlackenstoffe nachhaltig verdrängt und ausgeschieden werden.

Die DE-OS-196 22 708 beschreibt eine phytobiologische Zubereitung für die topische und parentarale, inkorporale Anwendung, zur Vorbeugung, Pflege und Behandlung verschiedener pathologischer und sonstiger endogener und exogener Einwirkungen, Veränderungen und Erkrankungen, sowie zur Erhaltung, Heilung und Wiederherstellung der Homöostase in und an tierischen und menschlichen Organen und Weichgeweben, dadurch gekennzeichnet, daß sie die folgenden Komponenten aufweisen:
(a) eine oder mehrere ionenhaltige Komponenten und Mineralsalze,
(b) eine oder mehrere adstringierende Komponenten, ein oder mehrere Bindemittel, ein Feuchtigkeitsmittel und ein oder mehrere etherische Öle, und
(c) gegebenenfalls ein oder mehrere pflanzliche Extrakte, ein Geliermittel, Säurebindungen, Hyaluronidase oder sonstige zweckentsprechende Komponenten in einem Mengenanteil zwischen 0,01 und 20 %. Diese Zubereitung kann gegebenenfalls Calendula, Hamamelis oder andere homöopathische Komponenten, Aminosäuren, Enzyme, Vitamine, Elektrolyte, Farbstoffe, Wachs, Emulgatoren, Stärke, Vaseline, Paraffine, Öle, Säuren, Fette, pflanzliche Mischungen und Extrakte, Harnstoff, Sulfatverbindungen etc. enthalten. Die Patentanmeldung nennt weder Beispiele für Aminosäuren noch werden in den Beispielen Aminosäuren verwendet.

Eine Aufgabe der vorliegenden Erfindung ist, ein Mittel zur Pflege und/oder Behandlung von Haut und Gewebe bereitzustellen, das sich durch rasche Wirkung, ausgezeichnete Verträglichkeit, breiten Anwendungsbereich und insbesondere große Tiefenwirkung auszeichnet. Insbesondere soll das Mittel zur Pflege, zum Schutz, zur Vorbeugung gewebsschädigender Manifestationen und Einwirkungen und zur Behandlung von Haut und Gewebe anwendbar sein. Das erfindungsgemäße Mittel soll Forschungsergebnisse zur Diffusion von Ionen durch Ionenkanäle in den intrazellulären Raum berücksichtigen und die Mikrozirkulation in der Zelle verbessern.

Eine Aufgabe der vorliegenden Erfindung wird durch die Bereitstellung einer Zusammensetzung gelöst, die folgende Komponenten umfaßt:
(a) mindestens ein Salz ausgewählt aus Alkali- und Erdalkalimetallsalzen und anderen Mineralstoffen,
   dadurch gekennzeichnet, daß es
(b) mindestens eine Aminosäure und
(c) Zinkoxid und/oder ein anorganisches Peroxid enthält.

Vorzugsweise wird als Bestandteil (c) der Zusammensetzung Zinkoxid, Magnesium- oder Natriumperoxid verwendet.

In bevorzugten Ausführungsformen kann das erfindungsgemäße Mittel zusätzlich jeweils unabhängig voneinander mindestens ein adstringierendes Mittel, ein Feuchtigkeitsmittel, ein etherisches Öl, Tego-Betain, sekundäre Pflanzenstoffe wie Flavonoide und Epigallocatechine, ungesättigte Fettsäuren, Liposomen, Vitamine, Spurenelemente und antimykotische und antimikrobische Komponenten umfassen. Ferner kann es übliche Träger- und Hilfsstoffe sowie Binde- und Haftmittel und übliche Lösungsmittel umfassen. Das Mittel der vorliegenden Erfindung wird in einer bevorzugten Ausführungsform topisch durch direkte Anwendung am Wirkort verwendet.

Das Mittel der vorliegenden Erfindung berücksichtigt neue Forschungsergebnisse zur Diffusion von Ionen durch Ionenkanäle in den intrazellulären Raum. Hierbei dringen Ionen aus Mineralsalzen durch die oberen Hautschichten hindurch bis tief ins Zellinnere von Unterhaut-, Bindegewebs- und Fettzellen ein. Das erfindungsgemäße Mittel verwendet unter anderem das Prinzip des Ionenaustausches zwischen Zellinnerem und Zelläußeren unter Verwendung von hohem osmotischen Druck, der durch die Wirkstoffkombination des Mittels aufgebaut wird. Eine entscheidende Rolle spielen dabei jedoch die Aminosäuren, mit deren Hilfe die Ionen durch sogenannte Ionenkanäle effektiver über die natürlichen Barrieren der Zellmembranen hinweg ins Zellinnere, dem eigentlichen Wirkort, gelangen.

Die erfindungsgemäße Zusammensetzung ist insbesondere auch dann noch wirksam, wenn die Funktionsfähigkeit der Zelle im Bereich der Ionentätigkeit eingeschränkt ist. Insbesondere wirken die Aminosäuren bei eingeschränkter Ionentätigkeit als zusätzlicher Carrier für Stoffe, die für das intrazelluläre Geschehen eingeschleust werden müssen, um hier funktional zu wirken. Es wurde gefunden, daß die Kombination aus Aminosäuren und Zinkoxid und/oder anorganischen Peroxiden die Mikrozirkulation in der Zelle verbessern im Vergleich zur Verwendung von Salzen. Die Verbesserung läßt sich visuell und biometrisch zeigen. Eine Erhöhung der Mikrozirkulation in der Zelle bei Verwendung von Salzen alleine geht nach ca. 1,5 Stunden abrupt auf den Ausgangswert zurück. Bei der erfindungsgemäßen Zusammensetzung, umfassend die Kombination aus Aminosäuren und Zinkoxid und/oder anorganischen Peroxiden, ist im Vergleich zu den Salzen die Verbesserung der Mikrozirkulation höher, kontinuierlicher, hält länger an und nähert sich langsam dem Ausgangswert. Dadurch wird eine bessere Einschleusung und Verteilung von Stoffen in die Zelle ermöglicht, wodurch der synergistische Effekt erhöht wird.

Ferner hat im Gegensatz zu den Salzen alleine eine Kombination von Salzen und Aminosäuren eine erhöhte positive Wirkung auf die Hautphysiologie. Hiervon sind insbesondere betroffen der Feuchtigkeitsfaktor (moisturing factor), der pH-Wert und die Sebometrie der Haut. Bei einer Anwendung von Salzen in Kombination mit Aminosäuren werden die Salze effektiver in die Zelle eingeschleust als beim Fehlen von Aminosäuren, da die Aminosäuren zum einen ein Schlüssel für die Zellmembranen sind und zum anderen die Aktivierung der Ionenkanäle unterstützen und erhöhen.

Alkali- und Erdalkalimetallsalze und andere Mineralstoffe sind lebensnotwendige Reglerstoffe im Stoffwechsel. In der vorliegenden Erfindung können alle bekannten Alkali- und Erdalkalimetallsalze und Mineralstoffe, die auch als Spurenelemente vorhanden sein können, verwendet werden. Bevorzugte, in der vorliegenden Erfindung verwendbare Vertreter aus der Gruppe der Alkali- und Erdalkalimetallsalze und Mineralstoffe sind Natrium, Kalium, Magnesium, Calcium, Silicium, Zink, Mangan, Kupfer, Eisen, Fluor, Chlor, Brom, Iod und Phosphor. Bevorzugte Spurenelemente sind die akzidentellen Spurenelemente, wie Silber, Gold, Aluminium, Barium, Wismut, Cadmium, Chrom, Nickel, Blei, Zinn, Titan und Vanadium, und die essentiellen Spurenelemente, wie Chrom, Kobalt, Kupfer, Fluor, Eisen, Iod, Mangan, Molybdän und Selen, die als Bestandteile in z.B. Enzymen, Chromoproteinen und Hormonen vorhanden sind. Der Anteil der Alkali- und Erdalkalimetallsalze und anderen Mineralstoffe im erfindungsgemäßen Mittel beträgt dabei bevorzugt 10 bis 90 Gew.-%, stärker bevorzugt 20 bis 85 Gew.-%, insbesondere bei topisch anwendbaren Mitteln 10 bis 30 Gew.-%, stärker bevorzugt 10 bis 25 Gew.-%, bezogen auf die Summe aller Bestandteile im Mittel, in Abhängigkeit vom erwünschten osmotischen Effekt. Die Alkali- und Erdalkalimetallsalze und die anderen Mineralstoffe werden in Form üblicher Salzverbindungen bzw. organischer Verbindungen zugegeben. Beispielsweise werden Natrium, Kalium und Magnesium bevorzugt in Form ihrer Chloridsalze, bzw. Natrium und Calcium in Form von Phosphaten zugegeben.

Die in der vorliegenden Erfindung gegebenenfalls verwendbaren adstringierende Mittel umfassen die zu diesem Zweck üblichen Verbindungen. Bevorzugt wird als Adstringent Tannin, Hamamelis, Rhabarber, Ratanhia und Salbei verwendet. Der Anteil der adstringierenden Mittel im erfindungsgemäßen Mittel beträgt dabei bevorzugt 0 bis 30 Gew.-%, stärker bevorzugt 1 bis 25 Gew.-%, bezogen auf die Summe aller Bestandteile im Mittel. Die adstringierenden Mittel werden dabei bevorzugt in reiner Form zugegeben.

In der vorliegenden Erfindung können gegebenenfalls übliche Feuchtigkeitsmittel verwendet werden. Bevorzugte Feuchtigkeitsmittel sind Glycerin, Aloe Vera, Kollagen, Desamidokollagen, Kollagenhydrolysate, Elastinhydrolysate, Hyalomuccolösung, Fibrostimulin, PN 73, Q 10, Wasser, Aloe Barbadensis Gel, Camelia Sinensis Extrakt, Efeu Extrakt (Hedera Helix), Matricaria (Kamille rekutita) Öl und Butylenglykol. Der Anteil der Feuchtigkeitsmittel im erfindungsgemäßen Mittel beträgt dabei bevorzugt 0 bis 70 Gew.-%, stärker bevorzugt 5 bis 50 Gew.-%, bezogen auf die Summe aller Bestandteile im Mittel.

Das Mittel der vorliegenden Erfindung kann ferner gegebenenfalls übliche etherische Öle umfassen. Bevorzugte etherische Öle sind dabei Öle aus Kamille, Lavendel, Rosmarin, Campfer, Latschenkiefern, Minzen, Teebaum und Eukalyptus. Der Anteil der etherischen Öle im Mittel der vorliegenden Erfindung beträgt dabei bevorzugt 0 bis 70 Gew.-%, stärker bevorzugt 3 bis 50 Gew.-%, bezogen auf die Summe aller Bestandteile im Mittel. Die etherischen Öle werden bevorzugt in reiner Form oder in Form von Extrakten oder kalt- und warmgepreßten Ölen zugegeben.

Das Mittel der vorliegenden Erfindung kann alle bekannten Aminosäuren und Aminosäurederivate enthalten. Bevorzugte Aminosäuren und Aminosäurederivate sind Alanin, Phenylalanin, Cystein, Cystin, Prolin, Tyrosin, Serin, Histidin, Glycin, Leucin, Isoleucin, Valin, Tryptophan, Arginin, Lysin, Asparagin und Glutamin. Insbesondere werden Cystin, Cystein, Prolin, Serin, Histidin, Lycin, Leucin, Isoleucin, Valin, Tyrosin, Arginin, Lysin, Asparagin und Glutamin verwendet. Besonders bevorzugt sind Cystin, Histidin, Glycin, Leucin, Valin, Arginin, Lysin und Glutamin. Von den Aminosäuren können die D-, die DL- und die L-Form verwendet werden, wobei die L-Form bevorzugt ist. Beispiele für Aminosäurederivate sind N-acetylierte Formen, z.B. N-Acetyl-L-glutamin, N-Acetyl-L-tyrosin und N-Acetyl-DL-tryptophan. Die Aminosäuren und Aminosäurederivate können einzeln oder in Form von Mischungen verwendet werden. Der Anteil an Aminosäuren und Aminosäurederivate im Mittel der vorliegenden Erfindung beträgt dabei bevorzugt 0,1 bis 40 Gew.-%, stärker bevorzugt 0,2 bis 30 Gew.-%, insbesondere bevorzugt 0,2 bis 15 Gew.-%, bezogen auf die Summe aller Bestandteile im Mittel. Die Aminosäuren und ihre Derivate werden bevorzugt in reiner Form zugegeben.

Die Mittel der vorliegenden Erfindung sind ferner gekennzeichnet durch die Anwesenheit von Zinkoxid und/oder einem anorganischen Peroxid. Als anorganische Peroxide werden dabei bevorzugt Zink-, Natrium-, Kalium-, Calcium- oder Magnesiumperoxid verwendet. Zinkoxid und/oder ein anorganisches Peroxid können beispielsweise zur Regelung des osmotischen Drucks verwendet werden. Die Kombination von Aminosäuren mit Zinkoxid und/oder einem anorganischen Peroxid weist überraschenderweise besonders gute Wirksamkeit gegenüber der alleinigen Verwendung von Magnesiumperoxid auf. Der Gesamtanteil von Zinkoxid und anorganischem Peroxid im Mittel der vorliegenden Erfindung beträgt dabei vorzugsweise 0,5 bis 50 Gew.-%, stärker bevorzugt 1,5 bis 40 Gew.-%, bezogen auf die Summe aller Bestandteile im Mittel. Der Anteil an anorganischem Peroxid sollte vorzugsweise bei topischer Anwendung nicht größer als 10 Gew.-%, stärker bevorzugt nicht größer als 6 Gew.-%, insbesondere nicht größer als 3 Gew.-%, und bei innerer Anwendung nicht größer als 20 Gew.-%, stärker bevorzugt nicht größer als 15 Gew.-%, sein. Zinkoxid und anorganisches Peroxid werden bevorzugt in reiner Form zugegeben.

Im Mittel der vorliegenden Erfindung kann zusätzlich gegebenenfalls Tego-Betain vorhanden sein. Der Anteil von Tego-Betain im erfindungsgemäßen Mittel beträgt dabei vorzugsweise 0 bis 25 Gew.-%, stärker bevorzugt 1 bis 20 Gew.-% und insbesondere bevorzugt 5 bis 10 Gew.-%, bezogen auf die Summe aller Bestandteile im Mittel.

Gegebenenfalls kann das Mittel der vorliegenden Erfindung alle bekannten bioaktiven Pflanzenstoffe, auch sekundäre Pflanzenstoffe genannt, enthalten. Die in der vorliegenden Erfindung verwendeten sekundären Pflanzensubstanzen umfassen insbesondere Carotinoide, Phytosterine, Saponine, Polyphenole, Flavonoide, Terpene, Phytoöstrogene, Sulfide, Phytinsäure und Ballaststoffe. Von den vorstehend genannten bioaktiven Pflanzensubstanzen werden in der vorliegenden Erfindung besonders die Polyphenole, die Flavonoide und Bioflavonoide verwendet. Besonders bevorzugt sind im erfindungsgemäßen Mittel Bioflavonoide natürlichen Ursprungs. Die bioaktiven Pflanzenstoffe können dabei in den bevorzugten Ausführungsformen des Mittels in einer bevorzugten Menge von 0 bis 75 Gew.-%, stärker bevorzugt 2 bis 50 Gew.-%, vorhanden sein, bezogen auf die Summe aller Bestandteile im Mittel. Bevorzugte natürliche Quellen für die Bioflavonoide sind Gemüse, wie Hülsenfrüchte, Karotten, Tomaten, Brokkoli und Paprika, Getreide, Sesam, Citrusfrüchte, Grüner und Schwarzer Tee, Johanniskraut, Trauben, etc. Die bioaktiven Pflanzensubstanzen werden bevorzugt in Form von Extrakten zugegeben.

Im Mittel der vorliegenden Erfindung können gegebenenfalls zusätzlich alle bekannten ungesättigten Fettsäuren verwendet werden. Es werden bevorzugt die in pflanzlichen Ölen und tierischen Ölen (wie Fischöl) enthaltenen ungesättigten Fettsäuren verwendet. Mehrfach ungesättigte Fettsäuren aus pflanzlichen Quellen sind essentielle Vorstufen von wichtigen Stoffwechselregulatoren (Eicosanoide und Prostaglandine). Die ungesättigten Fettsäuren sind dabei im erfindungsgemäßen Mittel bevorzugt in einem Anteil von 0 bis 70 Gew.-%, stärker bevorzugt 2 bis 45 Gew.-%, vorhanden, bezogen auf die Summe aller Bestandteile im Mittel. Beispiele für bevorzugte rein pflanzliche Quellen von ungesättigten Fettsäuren sind Nachtkerzen-, Lein-, Oliven-, Weizenkeim-, Soja-, Sonnenblumen-, Boretsch- und Kürbiskernöl und Öl aus dem Samen der roten Johannisbeere. Die ungesättigten Fettsäuren werden dabei bevorzugt in Form von kaltgepreßten Ölen zugegeben.

Das Mittel der vorliegenden Erfindung kann ferner gegebenenfalls zusätzlich übliche Liposomen, Lecithin und Lipodermin enthalten. Liposomen sind wichtig, da sie die Freigabe von Vitamin A und Vitamin E steuern. Dadurch sind diese Vitamine noch über einen längeren Zeitraum zugänglich. Der Anteil von Liposomen, Lecithin oder Lipodermin im erfindungsgemäßen Mittel beträgt dabei bevorzugt 0 bis 30 Gew.-%, stärker bevorzugt 2 bis 20 Gew.-%, bezogen auf die Summe aller Bestandteile im Mittel.

Das Mittel der vorliegenden Erfindung kann gegebenenfalls zusätzlich Epigallocatechine, vorzugsweise gewonnen aus Grünem Tee, umfassen. Durch Epigallocatechine kann die Zellalterung verzögert werden. Ferner sind sie wichtig als Cosubstanzen und dienen zur Unterstützung der Wirkung von Mikronährstoffen, wie den Vitaminen. Der Anteil der Epigallocatechine im Mittel beträgt dabei vorzugsweise 0 bis 60 Gew.-%, stärker bevorzugt 2 bis 30 Gew.-%, bezogen auf die Summe aller Bestandteile im Mittel. Die Epigallocatechine werden dabei bevorzugt in reiner Form oder als Extrakte zugegeben.

Das Mittel der vorliegenden Erfindung kann gegebenenfalls zusätzlich alle bekannten Vertreter der Vitamine und Provitamine umfassen. Insbesondere werden im Mittel bevorzugt die Vitamine A, die des B-Komplexes, C, D und E und β-Carotin verwendet. Der Anteil der Vitamine im Mittel der vorliegenden Erfindung beträgt vorzugsweise 0 bis 75 Gew.-%, stärker bevorzugt 5 bis 50 Gew.-%, bezogen auf die Summe aller Bestandteile im Mittel. Die Vitamine werden sowohl in natürlicher Form als Extrakte als auch synthetisch (z.B. B-Vitamine) zugesetzt, wobei Unterschiede in der Vitaminwirksamkeit damit nicht verbunden sind.

Gegebenenfalls kann das Mittel der vorliegenden Erfindung zusätzlich antimykotische und antimikrobische Komponenten umfassen. Dem erfindungsgemäßen Mittel können Antibiotika, Bakteriostatika, Corticosteroide, Cortisone, Econazolnitrat, Dexametason, Hydroxybenzoat, etc. zugegeben werden. Der Anteil der antimykotischen und antimikrobischen Komponenten im Mittel beträgt dabei vorzugsweise 0 bis 60 Gew.-% und insbesondere bevorzugt 2 bis 30 Gew.-%, bezogen auf die Summe aller Bestandteile im Mittel.

Gegebenenfalls kann das Mittel der vorliegenden Erfindung alle bekannten Hilfs-, Zusatz- und Trägerstoffe, die üblichen Binde- und Haftmittel und Lösungsmittel umfassen. Bevorzugte Beispiele sind dabei Milchfett, un-, teil- oder hydriertes Sojafett, Sojaöl, Walnußbutter, Glycerin, Gelatine, Pectin, Lecithin, β-Carotine, Sorbit-Lösung, Eisenoxid, Titandioxid, Farbstoffe, Fette, Wachse, Emulgatoren (z.B. IRICALMIN der Fa. Pentafarm Ltd., CH), Silikone, Polyethylene, Polysorbitone, (Meth)acryl-Verbindungen, Talkum, Dragantum, Xanthan-Gum, Stärke, Vaseline, Dextrose, Saccharin, Paraffine, Säuren, Konservierungs- und Duftstoffe. Als Binde- und Haftmittel kann z.B. Pectin verwendet werden. Die obengenannten Stoffe und Mittel werden dabei in üblicher Menge verwendet, beispielsweise Pectin bis zu 10 Gew.-%, bezogen auf die Summe aller Bestandteile im Mittel.

Die Herstellung des Mittels der vorliegenden Erfindung kann in für jedem Fachmann geläufiger Weise dadurch erfolgen, daß man beispielsweise die Wirkstoffe zusammen mit geeigneten, nichttoxischen, inerten, pharmazeutisch verträglichen festen oder flüssigen Trägermaterialien und gegebenenfalls den üblichen Zusatz- und Hilfsstoffen und Lösungsmitteln in eine gallenische Darreichungsform bringt. Verfahren zur Herstellung gallenischer Darreichungsformen, wie z.B. Salben und Cremen, sind beispielsweise beschrieben in H. Sucker, B. Fuchs, P. Speiser, "Pharmazeutische Technologie", 2. Aufl. (1991), Georg Thieme Verlag Stuttgart; R. Voigt, "Lehrbuch der pharmazeutischen Technologie", Thieme Verlag, 1976. Für das Mittel der vorliegenden Erfindung sind alle bekannten Applikationsformen möglich. Bevorzugte Applikationsformen sind Creme, Salbe, Paste, Emulsion, Lotion, Fluid, Lösung, Gel, Puder, Spray, Gelatine, Schaum und dgl. Applikationsformen zur inneren Anwendung sind z.B. Kapseln, Tabletten, Dragees, Trinklösungen und Injektionslösungen z.B. für subkutane Injektionen. Als Applikationsform ist auch eine Depotform möglich.

Für das Mittel der vorliegenden Erfindung sind prinzipiell alle bekannten Applikationswege möglich. Besonders bevorzugt ist die topische Applikation z.B. durch Aufbringen einer entsprechenden Applikationsform auf die Haut, z.B. durch Auftragen, Einreiben, Einmassieren, Aufsprühen, Auftupfen, etc. Es ist auch eine Applikation in Form von Salbenverbänden oder subkutaner Injektion möglich. Das Mittel der vorliegenden Erfindung kann ein oder mehrmals täglich und sowohl über kurze als auch längere Zeiträume angewendet werden. Dabei richtet sich jedoch die Tagesdosis und die Häufigkeit der Anwendung pro Tag nach der Rezeptur des einzelnen erfindungsgemäßen Mittels.

Die Mittel der vorliegenden Erfindung eignen sich in Abhängigkeit von der Rezeptur als Nahrungsergänzungsmittel, Kosmetika oder Arzneimittel, vorzugsweise als topisches Kosmetika oder als topisches Arzneimittel zur Anwendung auf Haut und Gewebe von Säugern, und werden beispielsweise zur Pflege, zum Schutz, zur Vorbeugung gewebsschädigender Manifestationen und Einwirkungen und zur Behandlung von Haut und Gewebe verwendet. In einer bevorzugten Ausführungsform sind sie in Abhängigkeit ihrer Rezeptur insbesondere anwendbar bei allen Hautirritationen (z.B. gestörte Hautphysiologie, Sonnenbrand), Cellulite, Falten, Akne, Herpes, Psoriasis, Neurodermitis, Ozonschäden, Verbrennungen, Verätzungen, zellulären Stoffwechselstörungen und sonstigen Veränderungen mit Akkumulationen von Gewebsflüssigkeit, Fett und anderen Zellprodukten und -abbauprodukten, wie z.B. Verdickungen, Ödeme, Hämatome, und sind ferner anwendbar beispielsweise bei Hämorrhoiden, Rheuma, Arthrose und Hautkrebs. Das Mittel ist auch auf Schleimhäute, z.B. im Magen- und Darmtrakt anwendbar.

Die vorliegende Erfindung wird anhand eines Beispiels weiter erläutert.

### Beispiel 1

Es wurde auf übliche Weise ein erfindungsgemäßes Mittel mit folgenden Bestandteilen hergestellt:

| | |
|---|---|
| Zinkoxid | 8 Gew.-% |
| Natriumperoxid | 3 Gew.-% |
| Natriumphosphat | 10 Gew.-%. |
| Calciumphosphat | 6 Gew.-% |
| Calciumchlorid | 5 Gew.-% |
| Arginin | 7 Gew.-% |
| Leucin | 8 Gew.-% |
| Asparagin | 5,5 Gew.-% |
| Valin | 2 Gew.-% |
| Hamamelis | 1 Gew.-% |
| Tannin | 3 Gew.-% |
| Pectin | 1 Gew.-% |
| Tego-Betain | 2 Gew.-% |
| Vitamin A | 1 Gew.-% |
| Vitamin E | 1,5 Gew.-% |
| β-Carotin | 0,5 Gew.-% |
| Kollagen | 1,5 Gew.-% |
| Aloe Vera | 2 Gew.-% |
| Olivenöl | 2 Gew.-% |
| Carotinoide | 2 Gew.-% |
| Gelatine | 1 Gew.-% |
| Liposomen | 2 Gew.-% |
| gereinigtes Wasser ad | 100 Gew.-% |

Die Zusammensetzung des obengenannten Beispiels wurde bei Probanden auf einem Bein aufgetragen. Auf dem anderen Bein wurden Kontrollsubstanzen (Plazebosubstanzen; Kontrollbein) aufgebracht. Die Ergebnisse der Untersuchungen lassen sich wie folgt zusammenfassen:

Als akute Reaktion der obengenannten erfindungsgemäßen Rezeptur wurde ein Anstieg der Mikrozirkulation festgestellt. Nach ca. 50 Minuten beobachtete man eine signifikante Verbesserung der Mikrozirkulation, wobei nach ca. 100 Minuten ein maximaler Anstieg erreicht wurde. Die signifikante Wirkphase wurde nach ca. 120 Minuten beobachtet. Am Kontrollbein wurden keine Veränderungen der Mikrozirkulation festgestellt. Als weitergehende Reaktion, hervorgerufen durch das Auftragen der erfindungsgemäßen Rezeptur, konnte eine Reduktion der Fettschicht am behandelten Bein beobachtet werden, während keine Veränderung am Kontrollbein erfolgte. Durch die Untersuchungen konnte gezeigt werden, daß durch das erfindungsgemäße Mittel die Mikrozirkulation signifikant verbessert und die Fettschicht reduziert werden kann.

### Beispiel 2

| | Anteil (Gew.-%) |
|---|---|
| Aspargin | 0,30 |
| Leucin | 0,20 |
| Valin | 0,30 |
| Arginin | 0,20 |
| Zinkperoxid | 0,50 |
| Calciumphosphat | 4,50 |
| Natriumphosphat | 3,50 |
| Zinkoxid transparent | 2,00 |
| Caliumchlorid | 4,00 |
| Magnesiumsulfat | 3,00 |
| dest. Wasser | 62,90 |
| Hamamelis | 2,50 |
| Tego-Betain | 2,00 |
| Sorbitol | 6,00 |
| Rosmarinöl | 0,30 |
| Menthol | 0,30 |
| grüner Tee pulvis | 1,00 |
| Efeu/Zinnkraut/Algen/Grüntee | 5,00 |
| Xanthan-Gum | 1,50 |

Auf übliche Weise wurde obiges erfindungsgemäßes Mittel hergestellt.

Zunächst wurde die Mikrozirkulation auf einem bestimmten Hautareal eines Probanden gemessen. Anschließend wurde die Rezeptur von Beispiel 2 auf diesem Hautareal leicht einmassiert. Dann wurde die Mikrozirkulation sofort und im weiteren in halbstündigem Abstand über 4 Stunden gemessen. Die Mikrozirkulation wurde hierbei in Flukseinheiten gemessen. Die Meßergebnisse wurden gegen die Zeit aufgetragen. Im Vergleich zu einem im Handel erhältlichen Produkt, das Salze und Magnesiumperoxid umfaßt und wie das erfindungsgemäße Mittel auf einem anderen Hautareal aufgetragen wurde, wurde festgestellt, daß die Mikrozirkulation bei Verwendung der erfindugsgemäßen Rezeptur, welche die Kombination von bestimmten Aminosäuren und Zinkoxid und -peroxid umfaßt, früher anstieg, länger einen höheres Niveau beibehielt und wesentlich später zum Ausgangswert abflachte. Die Verbesserung im Vergleich zum Handelsprodukt betrug zwischen 7 und 15 % bei den jeweiligen Meßpunkten. Die Ergebnisse zeigen die verbesserte systemische, synergistische Wirkung der erfindungsgemäßen Rezeptur zum Vergleichsprodukt.

### Beispiel 3

Es wurde auf übliche Weise ein erfindungsgemäßes Mittel mit folgenden Bestandteilen hergestellt:

| | Anteil (Gew.-%) |
|---|---|
| dest. Wasser | 71,15 |
| Calciumphosphat | 1,50 |
| Leucin | 0,20 |
| Valin | 0,30 |
| Arginin | 0,20 |
| Aspargin | 0,30 |
| Zinkperoxid | 0,25 |
| Natriumphosphat | 1,50 |
| Zinkoxid transparent | 3,00 |
| Karion FP flüssig | 4,00 |
| Neo Dragold liquid | 0,30 |
| Calciumchlorid | 1,50 |
| Magnesiumsulfat | 2,50 |
| grüner Tee pulvis | 2,00 |
| Ringelblume | 3,00 |
| Hamamelis | 2,50 |
| Johanniskraut | 3,00 |
| Fragrance 1677 | 0,30 |
| Teebaumöl Australisch | 1,00 |
| Xanthan-Gum | 1,50 |

### Beispiel 4

Es wurde auf übliche Weise ein erfindungsgemäßes Mittel mit folgenden Bestandteilen hergestellt:

| | Anteil (Gew.-%) |
|---|---|
| dest. Wasser | 66,70 |
| Zinkoxid transparent | 2,00 |
| Natriumphosphat | 3,00 |
| Aspargin | 0,05 |
| Calciumphosphat | 3,50 |
| Calciumchlorid | 3,50 |
| Leucin | 0,20 |
| Magnesiumsulfat | 3,00 |
| Zinkperoxid | 0,30 |
| Tego-Betain | 2,00 |
| Hamamelis | 2,50 |
| Sorbitol | 5,00 |
| YLANG-YLANGÖL II | 0,10 |
| Lotusduft - ether. Ölmischung | 0,10 |
| Menthol | 0,05 |
| grüner Tee pulvis | 0,50 |
| IRICALMIN | 3,00 |
| GLYCODERM | 3,00 |
| Xanthan-Gum | 1,50 |

In weiteren Anwendungen von unter die Erfindung fallenden Rezepturen wurden signifikante positive Wirkungen bezüglich der Alterung, Elastizität, Feuchtigkeit, Faltenbildung und Straffung der Haut beobachtet.

## Patentansprüche

1. Mittel zur topischen Anwendung, umfassend folgende Komponenten:
(a) mindestens ein Salz ausgewählt aus Alkalimetallsalzen, Erdalkällmetallsalzen und anderen Mineralstoffen,
(b) mindestens eine Aminosäure,
(c) Zinkoxid und ein anorganisches Peroxid,
(d) mindestens einen sekundären Pflanzenstoff, und
(e) gegebenenfalls mindestens eine ungesättigte Fettsäure,
wobei die sekundären Pflanzenstoffe ausgewählt sind aus Carotinoiden, Phytosterinen, Saponinen, Polyphenolen, Flavonoiden, Terpenen, Phytoöstrogenen, Sulfiden und Phytinsäure.

2. Mittel nach Anspruch 1,
**dadurch gekennzeichnet, daß** es
(e) mindestens eine mehrfach ungesättigte Fettsäure aus pflanzlichen Quellen umfaßt,

3. Mittel nach Anspruch 1 oder 2, **dadurch gekennzeichnet, dass** es zusätzlich mindestens eine der folgenden Komponenten umfaßt:
(a) Tego-Betain und/oder mindestens ein Epigallocatechin und/oder mindestens ein Spurenelement,
(b) mindestens ein Liposom und/oder mindestens ein Vitamin,
(c) mindestens ein adstringierendes Mittel und/oder mindestens ein Feuchtigkeitsmittel und/oder mindestens ein etherisches Öl,
(d) antimykotische und/oder antimikrobische Komponenten,
(e) mindestens einen Bestandteil ausgewählt aus Trägerstoffen, Hilfsstoffen, Zusatzstoffen, Bindemittel, Haftmittel und Lösungsmittel.

4. Mittel nach einem der Ansprüche 1 bis 3, umfassend folgende Komponenten:
(a) 10 bis 90 Gew.-% eines Salzes, ausgewählt aus Alkalimetallsalzen, Erdalkalimetallsalzen und anderen Mineralstoffen,
(b) 0,1 bis 40 Gew.-% einer Aminosäure und
(c) einen Gesamtanteil von Zinkoxid und anorganischem Peroxid von 0,5 bis 50 Gew.-%,
bezogen auf die Summe aller Bestandteile im Mittel.

5. Mittel nach einem der Ansprüche 1 bis 4, umfassend als Komponente
(a) mindestens ein Metallsalz ausgewählt aus Natrium; Kalium-, Magnesium-, Calcium-, Silicium-, Zink-, Mangan-, Kupfer- und Eisensalzen, und
(b) mindestens eine Aminosäure ausgewählt aus Cystin, Cystein, Prolin, Serin, Histidin, Glycin, Leucin, Isoleucin, Valin, Tyrosin, Arginin, Lysin, Asparagin und Glutamin.

6. Mittel nach einem der Ansprüche 1 bis 5, **dadurch gekennzeichnet, dass** das anorganische Peroxid Zink-, Natrium-, Kalium-, Calcium- oder Magnesiumperoxid ist.

7. Verwendung eines Mittels nach einem der Ansprüche 1 bis 6 als Kosmetika.

8. Verwendung eines Mittels nach einem der Ansprüche 1 bis 6 zur Herstellung eines Arzneimittels zur topischen Anwendung.

9. Verwendung eines Mittels nach einem der Ansprüche 1 bis 6 zur Herstellung eines Arzneimittels zur topischen Anwendung zur Behandlung von Hautirritationen, Sonnenbrand, Cellulite, Falten, Akne, Herpes, Neurodermitis, Ozonschäden, Verbrennungen, Verätzungen, Verdickungen, Ödemen, Hämatomen, Hämorrhoiden, Rheuma, Arthrose und Hautkrebs.

## Claims

1. Preparation for the topical application comprising the following components:
(a) at least one salt selected from alkali metal salts, alkaline earth metal salts and other minerals,
(b) at least one amino acid,
(c) zinc oxide and an inorganic peroxide,
(d) at least one secondary plant substance, and
(e) optionally at least one unsaturated fatty acid,
wherein the secondary plant substances are selected from carotenoids, phytosterols, saponines, polyphenols, flavonoids, terpenes, phytoestrogenes, sulfides and phytic acid.

2. Preparation according to claim 1, **characterized in that** it comprises
(e) at least one polyunsaturated fatty acid of vegetable sources.

3. Preparation according to claim 1 or 2, **characterized in that** it additionally comprises at least one of the following components:
(a) tego-betaine and/or at least one epigallocatechine and/or at least one trace element,
(b) at least one liposome and/or at least one vitamin,
(c) at least one adstringent agent and/or at least one humectant and/or at least one ethereal oil,
(d) antifungal and/or antimicrobial components,
(e) at least one component selected from carrier substances, adjuvants, additives, binding agents, adhesive agents and solvents.

4. Preparation according to any one of claims 1 to 3, comprising the following components:
(a) 10 to 90 wt.-% of a salt, selected from alkali metal salts, alkaline earth metal salts and other minerals,
(b) 0.1 to 40 wt.-% of an amino acid, and
(c) a total amount of zinc oxide and inorganic peroxide of 0.5 to 50 wt.-%, based on the sum of all components in the preparation.

5. Preparation according to any one of claims 1 to 4, comprising as a component:
(a) at least one metal salt selected from sodium, potassium, magnesium, calcium, silicon, zinc, manganese, copper and iron salts, and
(b) at least one amino acid selected from cystine, cysteine, proline, serine, histidine, glycine, leucine, isoleucine, valine, tyrosine, arginine, lysine, asparagine and glutamine.

6. Preparation according to any one of claims 1 to 5, **characterized in that** the inorganic peroxide is zinc peroxide, sodium peroxide, potassium peroxide, calcium peroxide or magnesium peroxide.

7. Use of a preparation according to any one of claims 1 to 6 as a cosmetic.

8. Use of a preparation according to any one of claims 1 to 6 for the preparation of a pharmaceutical composition for topical application.

9. Use of a preparation according to any one of claims 1 to 6 for the preparation of a pharmaceutical composition for topical application for the treatment of skin irritations, sun bum, cellulites, wrinkles, acne, herpes, neurodermatitis, ozone damage, bums, caustic burns, thickenings, edemas, hematomas, hemorrhoids, rheumatism, arthrosis and skin cancer.

## Revendications

1. Agent pour utilisation topique, comprenant les composants suivants :
(a) au moins un sel choisi parmi les sels de métal alcalin, les sels de métal alcalino-terreux et d'autres substances minérales,
(b) au moins un acide aminé,
(c) l'oxyde de zinc et un peroxyde inorganique,
(d) au moins une substance secondaire de plante, et
(e) le cas échéant, au moins un acide gras insaturé, ou les substances secondaires de plante sout choisi parmi les caroténoïdes, les phytostérines, les saponines, les polyphénols, les flavonoïdes, les terpènes, les phyto-oestrogènes, les sulfures et l'acide phytique.

2. Agent selon la revendication 1, **caractérisé en ce qu'**il comprend
(e) au moins un acide gras polyinsaturé de source végétale.

3. Agent selon la revendication 1 ou 2, **caractérisé en ce qu'**il comprend en outre, au moins un des composants suivants :
(a) la tégo-bétaïne et/ou au moins un épigallocatéchol et/ou au moins un oligoélément,
(b) au moins un liposome et/ou au moins une vitamine,
(c) au moins un agent astringent et/ou au moins un agent hydratant et/ou au moins une huile éthérée,
(d) des composants antimycotiques et/ou antimicrobiens,
(e) au moins un constituant choisi parmi des supports, des auxiliaires, des additifs, des liants, des adhérents et des solvants.

4. Agent selon l'une des revendications 1 à 3, comprenant les composants suivants :
(a) 10 à 90% en poids d'un sel, choisi parmi les sels de métal alcalin, les sels de métal alcalino-terreux et d'autres substances minérales,
(b) 0,1 à 40% en poids d'un acide aminé, et
(c) une quantité totale en oxyde de zinc et peroxyde inorganique de 0,5 à 50% en poids,
sur base de la somme de tous les constituants dans l'agent.

5. Agent selon l'une des revendications 1 à 4, comprenant comme composants :
(a) au moins un sel métallique, choisi parmi les sels de sodium, potassium, magnésium, calcium, silicium, zinc, manganèse, cuivre et fer, et
(b) au moins un acide aminé, choisi parmi la cystine, la cystéine, la proline, la sérine, l'histidine, la glycine, la leucine, l'isoleucine, la valine, la tyrosine, l'arginine, la lysine, l'asparagine et la glutamine.

6. Agent selon l'une des revendications 1 à 5, **caractérisé en ce que** le peroxyde inorganique est le peroxyde de zinc, de sodium, de potassium, de calcium ou de magnésium.

7. Utilisation d'un agent selon l'une des revendications 1 à 6, comme agent cosmétique.

8. Utilisation d'un agent selon l'une des revendications 1 à 6, pour la préparation d'un agent pharmaceutique pour utilisation topique.

9. Utilisation d'un agent selon l'une des revendications 1 à 6, pour la préparation d'un agent pharmaceutique pour utilisation topique, pour le traitement d'irritations de la peau, des brûlures solaires, de la cellulite, des rides, de l'acné, de l'herpès, de la neurodermite, de lésions dues à l'ozone, de brûlures, de brûlures chimiques, d'épaississements, d'oedèmes, d'hématomes, d'hémorroïdes, des rhumatismes, de l'arthrose et du cancer de la peau.
